(19) Europäisches Patentamt
European Patent Office
Office européen des brevets

(11) **EP 3 827 789 A1**

(12) **EUROPEAN PATENT APPLICATION**
published in accordance with Art. 153(4) EPC

(43) Date of publication:
**02.06.2021 Bulletin 2021/22**

(51) Int Cl.:
***A61F 2/60*** *(2006.01)*

(21) Application number: **19842319.6**

(22) Date of filing: **22.07.2019**

(86) International application number:
**PCT/JP2019/028689**

(87) International publication number:
**WO 2020/022277 (30.01.2020 Gazette 2020/05)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
**KH MA MD TN**

(30) Priority: **24.07.2018 JP 2018138800**

(71) Applicant: **Bridgestone Corporation**
**Chuo-Ku**
**Tokyo 104-8340 (JP)**

(72) Inventors:
• **ITOI Dyta**
**Tokyo 104-8340 (JP)**
• **KIWAKI Yukihiro**
**Tokyo 104-8340 (JP)**
• **ODAIRA Miho**
**Tokyo 104-8340 (JP)**

(74) Representative: **Oxley, Robin John George**
**Marks & Clerk LLP**
**15 Fetter Lane**
**London EC4A 1BW (GB)**

(54) **SOLE FOR ATHLETIC PROSTHETIC LEG**

(57) A sole which is attached to a ground contact region of an athletic prosthetic leg having a leaf-spring-like leg portion extending to a side of a toe via at least one curved portion, the ground contact region extending in an arc from the toe to a side of the curved portion is provided. The sole includes a bottom surface having a shape conforming to an extending shape of the ground contact region, and the bottom surface has at least one width direction land portion defined by a plurality of width direction grooves extending in a width direction of the sole. Moreover, the width direction land portion includes a chamfer at each of both end edges at border positions, respectively, between a contact patch and both side walls facing the plurality of width direction grooves.

FIG. 3A

EP 3 827 789 A1

**Description**

TECHNICAL FIELD

The present disclosure relates to a sole attached to a ground contact region of an athletic prosthetic leg, and in particular, relates to a sole of an athletic prosthetic leg which ensures a footprint area to a road surface while inhibiting occurrence of uneven wear.

BACKGROUND

Conventionally, a prosthetic leg for athletics (hereinafter, referred to as an athletic prosthetic leg or simply referred to as a prosthetic leg) having a leaf-spring-like leg portion which extends to a side of a toe via a curved portion and in which a ground contact region extends from the toe to a side of the curved portion in an arc has been well-known. To such an athletic prosthetic leg having the leaf-spring-like leg portion, generally, a sole which abuts a road surface is attached to a bottom surface of the ground contact region.

For example, Patent Literature 1 illustrates a sole attached to a lower surface of a curved leaf-spring-like athletic prosthetic leg to correspond to sporting events such as jogging or running. In other words, Patent Literature 1 discloses the sole in which a number of outsole portions each having a hexagonal contact patch are attached at a lower surface of the sole contacting a road surface.

CITATION LIST

Patent Literature

PTL 1: Japanese Patent Laid-Open No. 2016-150189

SUMMARY

(Technical Problem)

Here, accompanied with overall speed-up in athletics using the athletic prosthetic leg, to enable stable running, ensuring the footprint area to the road surface has been an object. While the footprint area is ensured at the hexagonal outsole portions in the sole disclosed in Patent Literature 1, there has been a problem that uneven wear occurs at the outsole portions accompanied with use.

An object of the present disclosure is to provide the sole of the athletic prosthetic leg which ensures the footprint area to the road surface while inhibiting occurrence of uneven wear.

(Solution to Problem)

The inventor earnestly studied about means which resolves the problem. In other words, reviewing the bottom surface of the sole of the athletic prosthetic leg in detail, the inventor newly found that the footprint area to the road surface can be ensured while uneven wear is inhibited by designing the shape of recesses and protrusions when the recesses and protrusions are formed by forming grooves on a sole bottom surface, and arrived at the present disclosure.

According to the present disclosure, there is provided a sole of an athletic prosthetic leg, the sole being configured to be attached to a ground contact region of the athletic prosthetic leg having a leaf-spring-like leg portion extending to a side of a toe via at least one curved portion, the ground contact region extending in an arc from the toe to a side of the curved portion, the sole including: a bottom surface having a shape conforming to an extending shape of the ground contact region, wherein the bottom surface has at least one width direction land portion defined by a plurality of width direction grooves extending in a width direction of the sole, and the width direction land portion includes a chamfer at each of both end edges at border positions, respectively, between a contact patch and both side walls facing the plurality of width direction grooves.

(Advantageous Effect)

According to the present disclosure, the sole of an athletic prosthetic leg which ensures the footprint area to the road surface while occurrence of uneven wear is inhibited can be provided.

BRIEF DESCRIPTION OF THE DRAWINGS

In the accompanying drawings:

FIG. 1 is a side view of an athletic prosthetic leg to which a sole according to one embodiment of the present disclosure is attached;
FIG. 2 is a drawing illustrating a pattern of a bottom surface of the sole according to one embodiment of the present disclosure;
FIG. 3A is a cross-sectional view taken along the line II-II of FIG. 2;
FIG. 3B is a drawing illustrating a variation of a shape of a chamfer;
FIG. 3C is a drawing illustrating a variation of the shape of the chamfer;
FIG. 4A is a drawing for explaining in stages movement of a leg portion and a ground contact form in a case where the athletic prosthetic leg is worn and a wearer executes straight running;
FIG. 4B is a drawing for explaining in stages the movement of the leg portion and the ground contact form in a case where the athletic prosthetic leg is worn and the wearer executes straight running;
FIG. 4C is a drawing for explaining in stages the movement of the leg portion and the ground contact form in a case where the athletic prosthetic leg is worn and the wearer executes straight running;

FIG. 4D is a drawing for explaining in stages the movement of the leg portion and the ground contact form in a case where the athletic prosthetic leg is worn and the wearer executes straight running;

FIG. 5 is a drawing for illustrating the pattern of the bottom surface of the sole according to another embodiment of the present disclosure;

FIG. 6A is a cross-sectional view taken along the line IV-IV of FIG. 5;

FIG. 6B is a drawing illustrating a variation of a shape of a sipe; and

FIG. 6C is a drawing illustrating a variation of the shape of the sipe.

## DETAILED DESCRIPTION

Hereinafter, with reference to the drawings, a sole of an athletic prosthetic leg according to the present disclosure (hereinafter, it is also referred to as a sole) will be explained in detail with illustration of embodiments thereof.

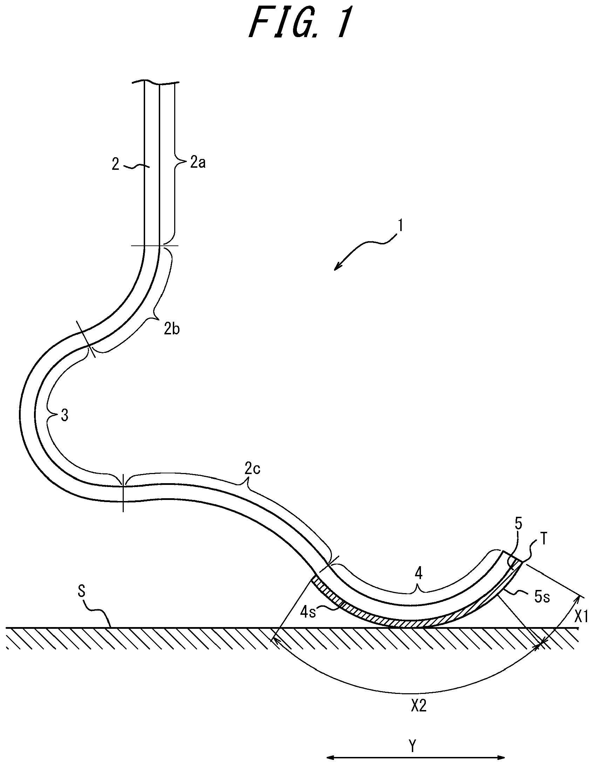

FIG. 1 is a side view of an athletic prosthetic leg 1 to which a sole 5 according to a first embodiment of the present disclosure is attached. The athletic prosthetic leg 1 has a leaf-spring-like leg portion 2, and the sole 5 is attached to a ground contact region at its tip side. Additionally, although not illustrated, a base end portion of the leg portion 2 is connected to a socket via an adapter and the socket houses a stump of a wearer's leg, whereby the wearer can wear the prosthetic leg. The adapter and the socket which correspond to the position of the stump of the leg, such as an above-knee prosthesis and a below-knee prosthesis, are used. FIG. 1 illustrates the leg portion 2 and the sole 5 in a standing state of the wearer who wears the athletic prosthetic leg 1.

Hereinafter, in this embodiment, in a height direction of the athletic prosthetic leg, a side where the leg portion 2 is connected to the adapter is referred to as a connection side, and a side where the leg portion 2 contacts a road surface S is referred to as a ground contact side. Also, a toe T of the athletic prosthetic leg 1 refers to a point at the forefront as a termination of the leg portion 2 extending from the connection side.

In this embodiment, the leg portion 2 of the athletic prosthetic leg 1 has a plate-like extending shape to the side of the toe T via at least one curved portion, in the illustrated example, one curved portion 3. In FIG. 1, the leg portion 2 is constituted by, in the order from the connection side to the ground contact side, a straight portion 2a, a curved portion 2b which is convex to the side of the toe T, the curved portion 3 which is convex to a rear side in a leg portion front-rear direction Y, a curved portion 2c which is concave to the ground contact side and a ground contact portion 4 which is convex to the ground contact side to extend to the side of the toe T in an arc.

Additionally, although the material of the leg portion 2 is not limited, from a viewpoint of strength and weight saving, fiber reinforced plastic etc. is preferably used.

The ground contact portion 4 includes a ground contact region 4s extending from the toe T to the side of the curved portion 3 in an arc at the ground contact side, and the sole 5 is attached to the ground contact region 4s. The ground contact region 4s refers to the entire region abutting the road surface S when the wearer who wears the athletic prosthetic leg 1 executes straight running movement, and in a state that the sole 5 is attached, the ground contact region 4s abuts the road surface S via the sole 5.

Although the material of the sole 5 is not limited, for example, rubber can be used.

The sole 5 has a shape conforming to an extending shape of the ground contact region 4s. Also, the ground contact side of the sole 5 is a sole bottom surface 5s. As illustrated in FIG. 1, the sole bottom surface 5s has a shape in which an arc X1 and an arc X2 are continued from the side of the toe T to the side of the curved portion 3. While the arc X1 and the arc X2 have a different radius of curvature to each other in this embodiment, they may include the same radius of curvature.

Hereinafter, a direction in which the sole bottom surface 5s extends from the side of the toe T to the side of the curved portion 3 is referred to as a Y direction, and a widthwise direction of the sole bottom surface 5s orthogonal to the Y direction is referred to as a W direction. Further, a thickness direction of the sole 5 is referred to as a Z direction.

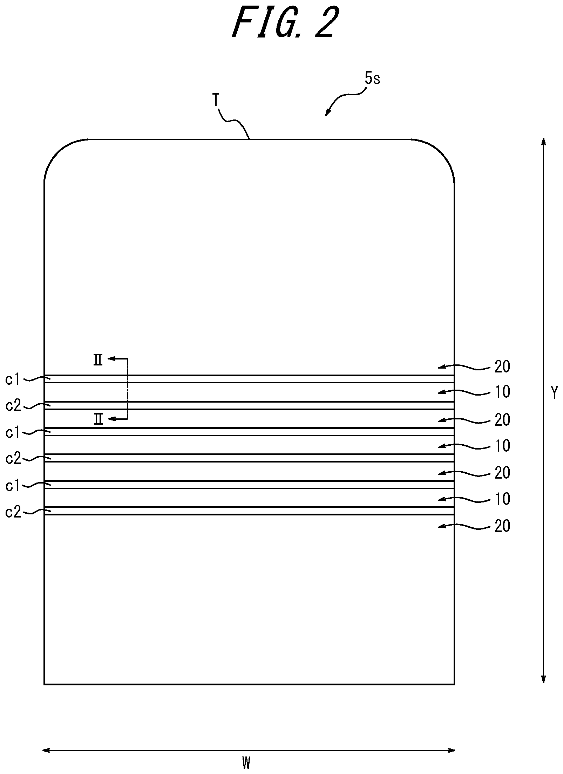

FIG. 2 is a drawing which illustrates a pattern of the sole bottom surface 5s according to one embodiment of the present disclosure. On the sole bottom surface 5s, at least one width direction land portion 10, in the illustrated example, three rows of width direction land portions 10 defined by a plurality of width direction grooves 20 extending in the W direction are arranged.

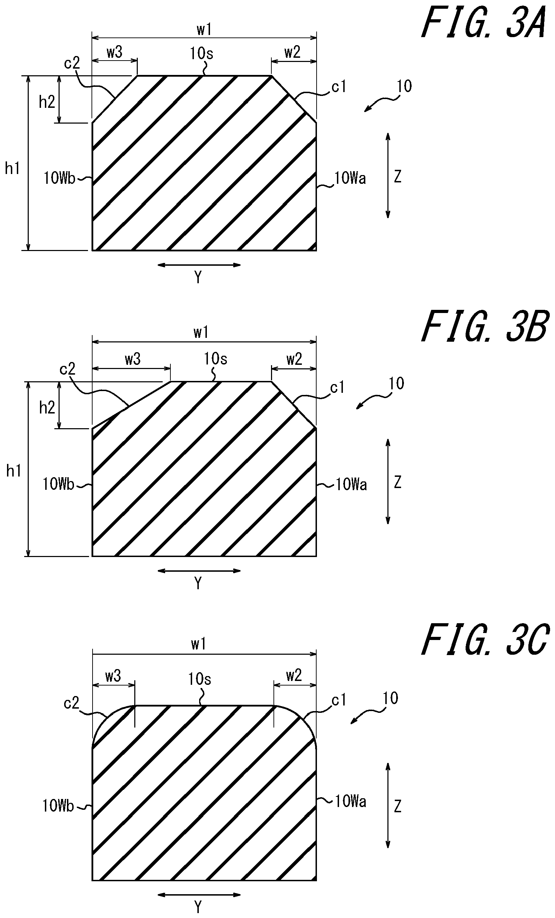

FIG. 3A is a section view which is orthogonal to an extending direction of the width direction land portion 10, that is, a cross-sectional view taken along the line II-II of FIG. 2. The width direction land portion 10 includes chamfers c1 and c2 at both end edges at border positions between a contact patch 10s and both side walls 10Wa and 10Wb facing the width direction grooves 20. In other words, the width direction land portion 10 has a shape in which chamfering is made at a corner of the side wall 10Wa and the contact patch 10s as well as a corner of the side wall 10Wb and the contact patch 10s. While the shape of the chamfers c1 and c2 is arbitrary, for example, a straight line as illustrated in FIGS. 3A and 3B and an arc having a center of a radius of curvature in an inner portion of the width direction land portion 10 as illustrated in FIG. 3C may be applied.

The width direction land portion 10 has the shape including the chamfers c1 and c2, whereby the following effects can be obtained. In other words, during running of the wearer of the prosthetic leg, at the time of movement in which the sole bottom surface 5s sequentially contacts the ground from the side of the curved portion 3 to the side of the toe T, or from the side of the toe T to the side of the curved portion 3, a ground contact pressure has been inclined to be higher at an end edge on the side of the road surface S of the width direction land portion 10. Especially, when the end edge includes an edge component, prior abrasion has been inclined to occur mainly at the edge component. Accordingly, by making a chamfered shape at both the end edges at the border positions between the contact patch 10s and both the side walls 10Wa and 10Wb facing the width direction grooves 20, occurrence of prior abrasion at both the end edges can be avoided. Also, since the width direction land portion 10 contacts the ground from the contact patch 10s and a portion including the chamfers c1 and c2 are pressed in the Z direction, occurrence of strain at the contact patch 10s due to a rubber flow to an outer side in the Y direction can be prevented. Consequently, ground contact pressure can be maintained as even and the footprint area can be fully ensured.

In the width direction land portion 10, a height h1 in the Z direction and a length w1 in a direction orthogonal to the extending direction of the width direction land portion 10 are preferably such that the height h1 is 1.5 times larger or more and 10.0 times larger or less with respect to the length w1. With this configuration, at the time of running of the wearer of the athletic prosthetic leg 1, a sufficient rigidity can be applied to the width direction land portion 10 allowing the sole bottom surface 5s to have an appropriate flexibility.

In the chamfers c1 and c2, a height h2 in the Z direction is preferably 25% or more and 67% or less of the height h1 in the Z direction of the width direction land portion 10. Rendering the height h2/the height h1 25% or more can effectively prevent occurrence of uneven wear, while rendering the height h2/the height h1 67% or less can apply the sufficient rigidity to the width direction land portion 10. Preferably, the height h2/the height h1 is 33% or more and 60% or less, and more preferably, 50%.

Further, in the width direction land portion 10, the length w1 in the direction orthogonal to the extending direction of the width direction land portion 10 and a total of the lengths w2 and w3 of the chamfers c1 and c2 at both the end edges in the direction orthogonal to the extending direction of the width direction land portion 10 preferably satisfy the relationship of the following formula:

$$(\text{Formula}) \quad 0.3 \leq [w1-(w2+w3)]/w1 \leq 0.9.$$

By rendering a value of [w1-(w2+w3)]/w1 0.3 times larger or more, the footprint area at a moment of the first ground contact of the width direction land portion 10 can be fully ensured, which improves anti-slip performance. On the other hand, by rendering this value 0.9 times larger or less, drainage performance by the stepwise side walls can be exerted more efficiently.

More preferably, $0.3 \leq [w1-(w2+w3)]/w1 \leq 0.8$, and more preferably, $0.47 \leq [w1-(w2+w3)]/w1 \leq 0.78$.

Additionally, while the lengths w2 and w3 have the same length in FIG. 3A, the lengths w2 and w3 may be different as illustrated in FIG. 3B. In this case, preferably, the length w3 is larger than the length w2. In other words, by making the length w3 of the chamfer c2 located at the side of the curved portion 3 of the width direction land portion 10 larger than the length w2 of the chamfer c1 located at the side of the toe T, a side firstly abutting the road surface S has a shape which is relatively difficult to be collapsed when the sole bottom surface 5s contacts the ground from the side of the curved portion 3 to the side of the toe T. Consequently, when the width direction land portion 10 is pressed from the contact patch 10s to the Z direction, occurrence of strain can be efficiently inhibited, and thus the footprint area with the road surface S can be ensured more reliably.

Also, in the chamfers c1 and c2, the lengths w2 and w3 in the direction orthogonal to the extending direction of the width direction land portion 10 preferably have a length of 0.5 times larger or more and 8.0 times larger or less with respect to the height h2. With this configuration, by forming the chamfers c1 and c2, rigidity of the width direction land portion 10 is not deteriorated, and also the footprint area to the road surface can be efficiently ensured while occurrence of uneven wear can be inhibited.

Additionally, the length w1 of the width direction land portion 10 is preferably 2.0 mm or more and 8.0 mm or less. The length w1 of 2.0 mm or more applies sufficient land portion rigidity to the width direction land portion 10, while the length w1 of 8.0 mm or less can maintain flexibility of the sole bottom surface 5s in the Y direction. Preferably, the length w1 is 3.0 mm or more and 6.5 mm or less, and more preferably, 3.8 mm or more and 4.5 mm or less.

Also, the lengths w2 and w3 of the chamfers c1 and c2 are preferably 0.5 mm or more and 2.5 mm or less. The lengths w2 and w3 of 0.5 mm or more can improve an inhibition effect of uneven wear, while the lengths w2 and w3 of 2.5 mm or less fully ensure the footprint area without deteriorating rigidity of the width direction land portion 10.

Further, the height h1 of the width direction land portion 10 is preferably 1.0 mm or more and 7.0 mm or less. The height h1 of 1.0 mm or more can improve drainage performance due to the recesses and protrusions of the sole bottom surface 5s, while the height h1 of 7.0 mm or less can maintain rigidity of the width direction land portion 10. Preferably, the height h1 is 2.0 mm or more and 3.5 mm or less, and more preferably, 2.4 mm or more and 3.5 mm or less.

The height h2 of the chamfers c1 and c2 are preferably 0.5 mm or more and 2.5 mm or less. The height h2 of 0.5 mm or more can more effectively inhibit occurrence of uneven wear, while the height h2 of 2.5 mm or less can inhibit deterioration of rigidity of the width direction land portion 10. Preferably, the height h2 is 0.7 mm or more and 1.5 mm or less, and more preferably, 1.2 mm or more and 1.5 mm or less.

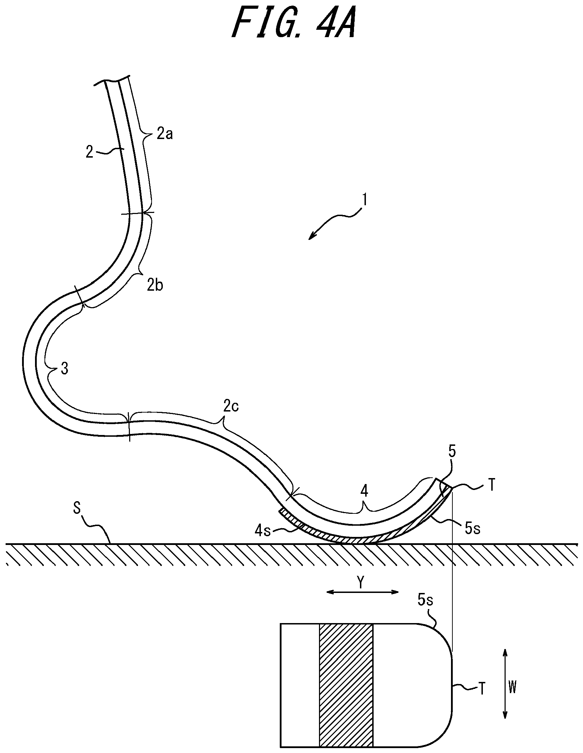

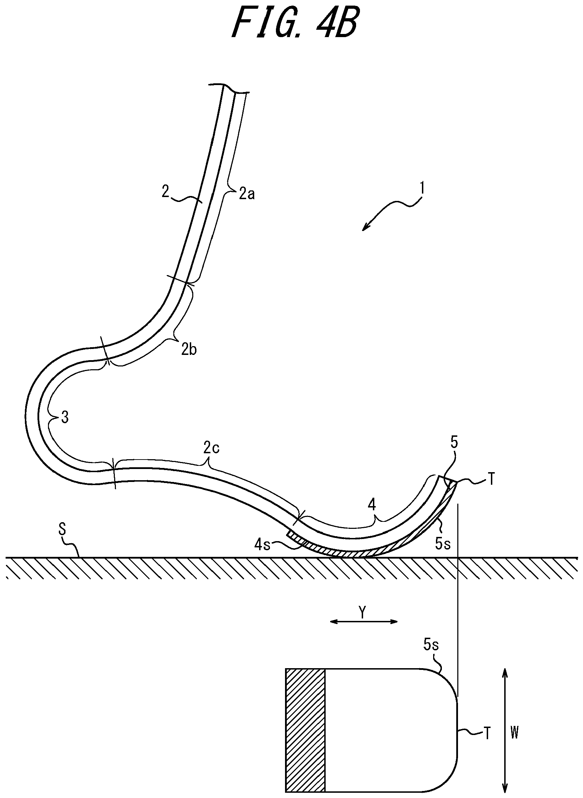

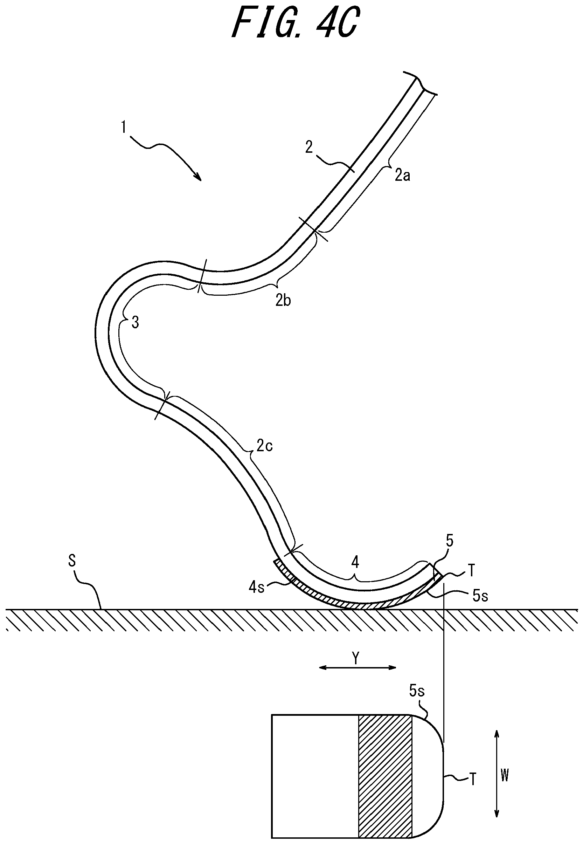

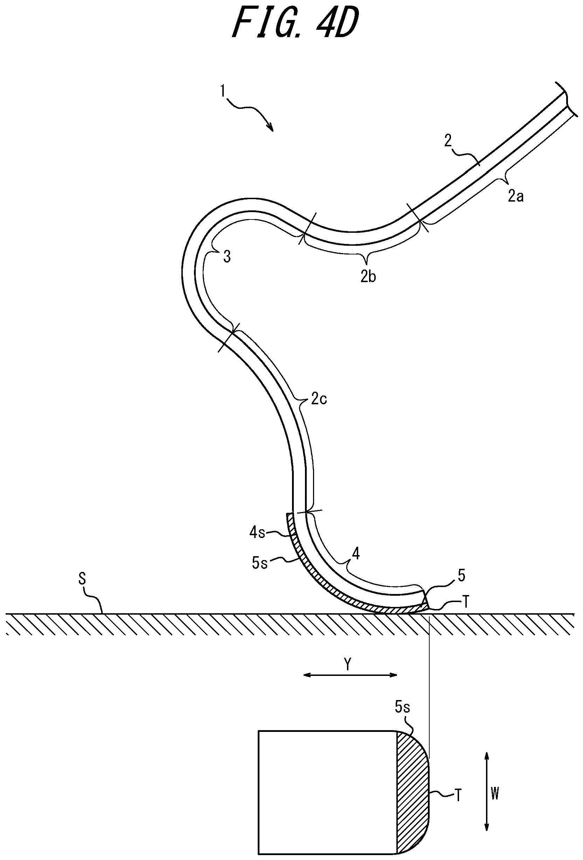

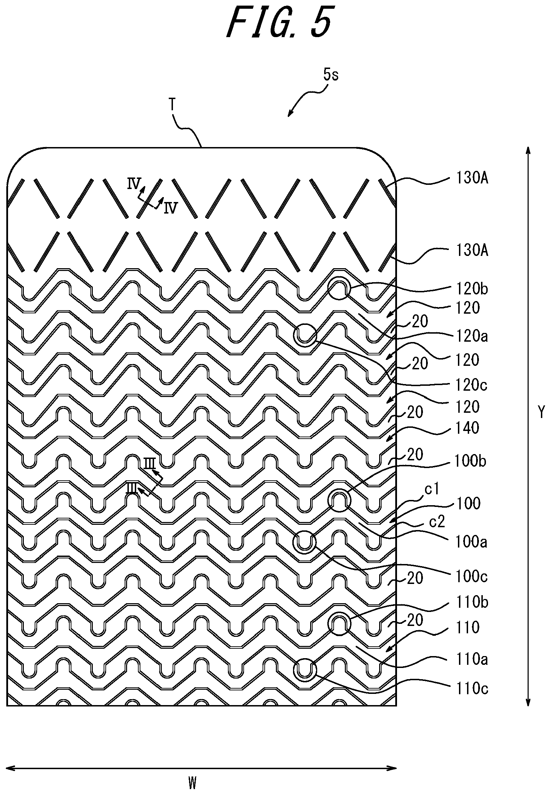

Next, with reference to FIGS. 4A, 4B, 4C, 4D and FIG. 5, a groove pattern of the bottom surface 5s of the sole of an athletic prosthetic leg according to another embodiment will be explained. Additionally, the sole of an athletic prosthetic leg according to a second embodiment has the same basic configuration as in the first embodiment, and the width direction land portions having the chamfers are formed. However, an extending shape of the width direction land portions is different from that in the first embodiment.

The pattern of the sole bottom surface 5s of the sole of an athletic prosthetic leg according to the second embodiment is based on a finding related to a ground contact form obtained from an experiment which will be explained later. An experiment result of the ground contact form of the sole bottom surface 5s will be explained using FIGS. 4A, 4B, 4C and 4D. FIGS. 4A, 4B, 4C and 4D are drawings for explaining in stages movement of the leg portion 2 and the ground contact form of the sole bottom surface 5s when the wearer who wears the athletic prosthetic leg 1 executes straight running. In each drawing, an upper portion is a side view of the leg portion 2 and the sole 5, and a lower portion illustrates a transition of the ground contact form of the sole bottom surface 5s when the wearer who wears the athletic prosthetic leg 1 executes straight running movement.

FIG. 4A illustrates a state that the wearer lowers the raised athletic prosthetic leg 1 to the road surface S and the entire weight is loaded on the athletic prosthetic leg 1. As illustrated in the lower portion of the drawing, at an initial stage in which the prosthetic leg 1 is lowered to the road surface S, the ground contact region of the sole bottom surface 5s is a region which is near a center portion of the bottom surface and is spaced in the Y direction from an end portion at the side of the curved portion 3 as well as the toe T of the sole bottom surface 5s.

FIG. 4B illustrates a state that the wearer steps forward, from the state of FIG. 4A, while the entire weight remains to be loaded on the athletic prosthetic leg 1. In a case of running of a healthy person, such a step form is generally applied that ground contact is sequentially executed from a heel side of a shoe sole which firstly contacts the ground toward the side of the toe, while in the athletic prosthetic leg 1, the ground contact region is moved to the side of the curved portion 3 from a region which firstly contacts the ground.

FIG. 4C illustrates a state that the wearer starts a kick-out movement of the athletic prosthetic leg 1 by shaking an opposite leg from the leg wearing the athletic prosthetic leg 1 forward. Entering into this kick-out movement, in the athletic prosthetic leg 1, a region at the side of the toe T from the regions illustrated in FIGS. 4A and 4B of the sole bottom surface 5s contacts the ground.

FIG. 4D illustrates a state that the wearer is in a final stage of kicking out the athletic prosthetic leg 1 just before separating from the road surface S. To kick out from the toe T of the sole bottom surface 5s, ground contact is executed further at a region at the side of the toe T than in FIG. 4C.

Based on the experiment result illustrated in FIGS. 4A, 4B, 4C and 4D, the inventor obtained the finding that separating functions of the sole bottom surface 5s in accordance with the transition of the ground contact region is advantageous to property improvement of the sole of the prosthetic leg, and conceived the following groove pattern.

In other words, in FIG. 5, the sole bottom surface 5s includes width direction land portions 100, 110, 120, 140 which are defined by width direction grooves 20 and extend in the width direction W and a sipe 130A. Additionally, a cross-sectional view taken along the line III-III of FIG. 5 corresponds to a cross-sectional shape of the width direction land portion 10 in FIG. 3A, and the width direction land portion 100 includes the chamfers c1 and c2 in the same manner as the width direction land portion 10. Also in the width direction land portions 110, 120 and 140, portions indicated by double lines in FIG. 5 are the chamfers.

The width direction land portion 100 is shaped to include a width direction extending portion 100a extending in the W direction to be substantially zigzag-shaped, a toe side protruding portion 100b extending to the side of the toe T from a bent portion bending to be convex to the side of the toe T and a curved portion side protruding portion 100c extending to the side of the curved portion 3 from a bent portion bending to be convex to the side of the curved portion 3. The width direction land portion 110 is shaped to include a width direction extending portion 110a, a toe side protruding portion 110b and a curved portion side protruding portion 110c. Additionally, the width direction land portion 140 has the same shape as the width direction land portion 110.

Here, a region where the width direction land portions 100 and 110 are arranged is one corresponding to the ground contact region illustrated in FIGS. 4A and 4B, in which the wearer firstly contacts the ground and executes a step movement in a state that the entire weight is loaded to the athletic prosthetic leg 1. Consequently, it is vital that this region fully grips the road surface S such that the entire body is balanced even when the entire weight of the wearer is loaded on the athletic prosthetic leg 1. The width direction extending portion 110a is allowed to be zig-zag shaped, and further, the toe side protruding portion 100b and the curved portion side protruding portion 100c are formed, whereby a drainage function is efficiently exerted along the Y direction and a gripping force with the road surface S is fully ensured. This inhibits slip of the prosthetic leg, that is, anti-slip performance can be improved.

Additionally, a land portion width of the width direction land portion 110 is larger than that of the width direction land portion 100. As illustrated in FIG. 4B, in a state of stepping immediately after the ground contact, the ground contact region is changed to the side of the curved portion 3 from the region which firstly contacts the ground, that is, at the opposite side from a direction that the wearer advances. Here, movement of an upper body in which the wearer tries to move forward and a direction of the change of the ground contact region are temporarily opposite, so that this is a stage requiring a high propulsive force for the kick-out movement at the latter half of the ground contact form as illustrated in FIGS. 4C to 4D. A higher land portion ratio is obtained by arranging, at the side of curved portion 3 from the width direction land portion 100, the width direction land portion 110 which has a larger width than the width direction land portion 100, and accordingly, rigidity of this arrangement region is improved and a high propulsive force can be achieved such that the step movement is smoothly continued to the kick-out movement.

The width direction land portion 120 is shaped to include a width direction extending portion 120a which extends in the W direction to be substantially zigzag-shaped and in which a land portion width is repeatedly wide and narrow, a toe side protruding portion 120b extending to be convex in an extending direction of the width direction extending portion 120a from a bent portion bending to be convex at the side of the toe T and a curved portion side protruding portion 120c extending to be convex in the extending direction of the width direction land portion 120a from a bent portion bending to be convex at the side of the curved portion 3.

A region where the width direction land portion 120 is arranged is one illustrated in FIG. 4C, which contacts the ground when the kick-out movement of the athletic prosthetic leg 1 is started. Application of the shape with the varied land portion width improves drainage performance while land portion rigidity is made higher. In other words, application of the shape in which a portion with a relatively large land portion width and a portion with a relatively small land portion width coexist avoids slipperiness due to enlargement of the land portion width, although the land portion width is enlarged.

Also, the sole bottom surface 5s of this embodiment preferably includes a plurality of sipes 130A each extending linearly in a direction which is inclined to the W direction at a region from an end edge at the side of the toe T toward the side of the curved portion 3. The region where the sipes 130A are arranged is one illustrated in FIG. 4D, in which the wearer shakes the opposite leg from the leg wearing the athletic prosthetic leg 1 forward to execute a kick-out movement of the athletic prosthetic leg 1. This region is one which easily develops abrasion in particular, because the ground contact is sequentially executed toward the toe T, and the wearer presses the road surface S by the sole bottom surface 5s to slidingly contact the ground. Consequently, in this region, it is vital that wear resistance performance is improved and anti-slip property is maintained.

Especially, a certain degree of region from the end edge of the sole bottom surface 5s at the side of the toe T where the sipes 130A are formed is a region corresponding to the arc X1 which continues with a certain radius of curvature from the toe T. As illustrated in FIG. 4D, this region finally contacts the ground when the wearer wearing the athletic prosthetic leg 1 executes the kick-out movement, so that a severer abrasion has been inclined to occur. Accordingly, this region needs to have an especially high wear resistance performance. Consequently, the sipes 130A each having a smaller width than the grooves formed in other regions are formed, so that an edge component is distributed without deteriorating rigidity. Due to this, the sole 5 is protected from severe abrasion and the long service life of the leg portion 2 itself can be achieved.

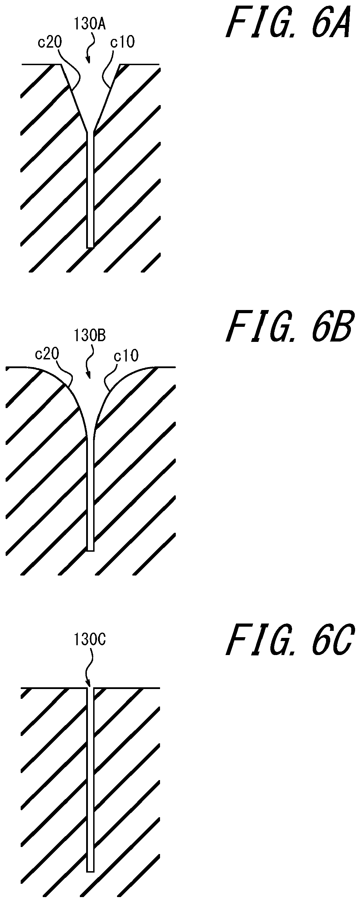

Also, preferably, the sipes 130A terminate in the sole bottom surface 5s, and as illustrated in FIG. 6A which is a cross-sectional view taken along the line IV-IV of FIG. 5, have the shape including chamfers c10 and c20 at both end edges at border positions between the sole bottom surface 5s and both side walls defining each sipe 130A. By providing the chamfers c10 and c20 at each sipe 130A, drainage performance of the sole bottom surface 5s can be improved, and further, occurrence of chipping and abrasion at each end edge at the side of the road surface S of the sipe 130A can be prevented.

Additionally, the shape of the sipes is arbitrary, and in addition to the shape of the sipe 130A illustrated in FIG. 6A, the chamfers c10 and c20 may be arcuate as in a sipe 130B illustrated in FIG. 6B. Further, as illustrated in FIG. 6C, the shape with no chamfer may be applied.

Additionally, in any of the embodiments described above, in the pattern of the sole bottom surface 5s, fluorine is preferably applied to a groove wall and a groove bottom constituting the width direction groove which defines width direction land portions. Since the fluorine is applied to the groove wall and the groove bottom of the width direction groove, drainage performance in the sole bottom surface 5s can be improved.

REFERENCE SIGNS LIST

    1 athletic prosthetic leg
    2 leg portion
    2a straight portion
    2b, 2c curved portion
    3 curved portion
    4 ground contact portion
    4s ground contact region
    5 sole
    5s sole bottom surface
    10 width direction land portion
    10s contact patch
    10Wa, 10Wb side wall

20 width direction groove

100, 110, 120, 140 width direction land portion

100a, 110a, 120a width direction extending portion

100b, 110b, 120b toe side protruding portion

100c, 110c, 120c curved portion side protruding portion

130A, 130B, 130C sipe

c1, c2, c10, c20 chamfer

w1 length of a width direction land portion 10 in a direction orthogonal to an extending direction

w2, w3 lengths of chamfers c1 and c2 in a direction orthogonal to the extending direction of the width direction land portion 10

h1 height of the width direction land portion 10 in a Z direction

h2 height of the chamfers c1 and c2 in the Z direction

S road surface

Y direction in which a sole bottom surface 5s extends from a side of a toe T to a side of a curved portion 3

W width direction of the sole bottom surface 5s which is orthogonal to a Y direction

Z thick direction of a sole 5

## Claims

1. A sole of an athletic prosthetic leg, the sole being configured to be attached to a ground contact region of the athletic prosthetic leg having a leaf-spring-like leg portion extending to a side of a toe via at least one curved portion, the ground contact region extending in an arc from the toe to a side of the curved portion, the sole comprising:

   a bottom surface having a shape conforming to an extending shape of the ground contact region,
   wherein the bottom surface has at least one width direction land portion defined by a plurality of width direction grooves extending in a width direction of the sole, and
   the width direction land portion includes a chamfer at each of both end edges at border positions, respectively, between a contact patch and both side walls facing the plurality of width direction grooves.

2. The sole of the athletic prosthetic leg according to claim 1, wherein a height of the chamfer in a thickness direction of the sole is 25% or more and 67% or less of a height of the width direction land portion in the thickness direction of the sole.

3. The sole of the athletic prosthetic leg according to claim 1 or 2, wherein a total of a length w2 and a length w3 of the chamfers at both the end edges in a direction orthogonal to an extending direction of the width direction land portion and a length w1 of the width direction land portion in a direction orthogonal to the extending direction of the width direction land portion satisfy a relationship of the following formula:

$$(\text{Formula}) \quad 0.3 \leq [w1 - (w2 + w3)]/w1 \leq 0.9.$$

4. The sole of the athletic prosthetic leg according to any one of claims 1 to 3, wherein the bottom surface includes at least one sipe extending linearly in a portion which continues with a constant radius of curvature from the toe, and
   the sipe terminates in the bottom surface and includes a chamfer at each of both the end edges at border positions, respectively, between the bottom surface and both the side walls defining the sipe, both the side walls extending in a longitudinal direction of the sipe.

# FIG. 1

# FIG. 2

*FIG. 3A*

*FIG. 3B*

*FIG. 3C*

EP 3 827 789 A1

# FIG. 4A

11

# FIG. 4B

# FIG. 4C

# FIG. 4D

# FIG. 5

*FIG. 6A*

c20   130A   c10

*FIG. 6B*

c20   130B   c10

*FIG. 6C*

130C

## INTERNATIONAL SEARCH REPORT

| International application No. |
| --- |
| PCT/JP2019/028689 |

A. CLASSIFICATION OF SUBJECT MATTER
Int.Cl. A61F2/60(2006.01)i

According to International Patent Classification (IPC) or to both national classification and IPC

B. FIELDS SEARCHED

Minimum documentation searched (classification system followed by classification symbols)
Int.Cl. A61F2/60, A43B13/22

Documentation searched other than minimum documentation to the extent that such documents are included in the fields searched
Published examined utility model applications of Japan    1922–1996
Published unexamined utility model applications of Japan    1971–2019
Registered utility model specifications of Japan    1996–2019
Published registered utility model applications of Japan    1994–2019

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
| --- | --- | --- |
| Y<br>A | US 8535390 B1 (LECOMTE, C. et al.) 17 September 2013, column 1, lines 18-21, column 2, line 46 to column 5, line 20, fig. 1A-5D<br>(Family: none) | 1-2, 4<br>3 |
| Y<br>A | JP 2013-66709 A (PARK, J. W.) 18 April 2013, paragraphs [0001], [0007]-[0010], [0027]-[0056], fig. 1-10<br>& US 2013/0074370 A1, paragraphs [0002], [0010]-[0013], [0056]-[0085], fig. 1-10 & KR 10-1167702 B1 | 1-2, 4<br>3 |

☒   Further documents are listed in the continuation of Box C.     ☐   See patent family annex.

| *   Special categories of cited documents: | "T"   later document published after the international filing date or priority date and not in conflict with the application but cited to understand the principle or theory underlying the invention |
| --- | --- |
| "A"   document defining the general state of the art which is not considered to be of particular relevance | |
| "E"   earlier application or patent but published on or after the international filing date | "X"   document of particular relevance; the claimed invention cannot be considered novel or cannot be considered to involve an inventive step when the document is taken alone |
| "L"   document which may throw doubts on priority claim(s) or which is cited to establish the publication date of another citation or other special reason (as specified) | "Y"   document of particular relevance; the claimed invention cannot be considered to involve an inventive step when the document is combined with one or more other such documents, such combination being obvious to a person skilled in the art |
| "O"   document referring to an oral disclosure, use, exhibition or other means | |
| "P"   document published prior to the international filing date but later than the priority date claimed | "&"   document member of the same patent family |

| Date of the actual completion of the international search | Date of mailing of the international search report |
| --- | --- |
| 14.08.2019 | 27.08.2019 |

| Name and mailing address of the ISA/<br>Japan Patent Office<br>3-4-3, Kasumigaseki, Chiyoda-ku,<br>Tokyo 100-8915, Japan | Authorized officer<br><br>Telephone No. |
| --- | --- |

Form PCT/ISA/210 (second sheet) (January 2015)

**INTERNATIONAL SEARCH REPORT**

International application No.

PCT/JP2019/028689

C (Continuation).   DOCUMENTS CONSIDERED TO BE RELEVANT

| Category* | Citation of document, with indication, where appropriate, of the relevant passages | Relevant to claim No. |
|---|---|---|
| Y<br>A | JP 2009-247823 A (NOSAKKUSU KK) 29 October 2009, paragraphs [0001], [0006], [0033]-[0055], fig. 1-8 (Family: none) | 1-2, 4<br>3 |
| A | JP 2017-515519 A (OTTO BOCK HEALTHCARE GMBH) 15 June 2017, entire text, all drawings<br>& US 2017/0049585 A1 & WO 2015/172767 A1 | 1-4 |
| A | WO 2016/199402 A1 (BRIDGESTONE CORPORATION) 15 December 2016, paragraph [0033], fig. 7-9<br>& JP 2017-1468 A & US 2018/0162166 A1, paragraph [0052], fig. 7-9 & EP 3305552 A1 & CN 107614288 A | 2, 4 |
| A | JP 2017-197109 A (BRIDGESTONE CORPORATION) 02 November 2017, entire text, all drawings<br>& US 2019/0118584 A1 & WO 2017/187834 A1 & EP 3450212 A1 & CN 109070654 A | 4 |
| A | JP 2016-55814 A (BRIDGESTONE CORPORATION) 21 April 2016, entire text, all drawings<br>(Family: none) | 4 |

Form PCT/ISA/210 (continuation of second sheet) (January 2015)

**EP 3 827 789 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2016150189 A